# FASCICULE DE BREVET EUROPEEN

(11) **EP 0 600 536 B1**
(45) Date de publication et mention de la délivrance du brevet: **11.03.1998**
(21) Numéro de dépôt: 93203257.6
(22) Date de dépôt: 22.11.1993
(51) Int. Cl.: C07C 17/38, C07C 19/08

(54) **Procédé de purification de 1,1-difluoroéthane**
Verfahren zur Reinigung von 1,1-Difluorethan
Process for the purification of 1,1-difluoroethane

(30) Priorité: 02.12.1992 BE 9201057
(43) Date de publication de la demande: 08.06.1994
(73) Titulaire: SOLVAY (Société Anonyme), B-1050 Bruxelles (BE)
(72) Inventeur: Pennetreau, Pascal, B-1330 Rixensart (BE)
(74) Mandataire: Jacques, Philippe

(56) Documents cités:
- EP-A- 0 370 688
- EP-A- 0 433 129
- DE-A- 2 215 019

## Description

La présente invention se rapporte à la purification du 1,1-difluoroéthane. Elle concerne plus particulièrement un procédé de purification de 1,1-difluoroéthane brut en chlorure de vinyle.

Le 1,1-difluoroéthane présente un intérêt particulier comme substitut de certains chlorofluorocarbures, en particulier comme substitut du dichlorodifluorométhane (CFC-12).

Le 1,1-difluoroéthane peut être préparé par réaction de chlorure de vinyle avec du fluorure d'hydrogène, tel qu'il est décrit dans le brevet BE-766395 au nom de SOLVAY. Dans un tel procédé, à la sortie du réacteur d'hydrofluoration, le mélange de produits réactionnels contient, outre du 1,1-difluoroéthane, du chlorure d'hydrogène provenant de l'élimination de l'atome de chlore du composé de départ, du chlorure de vinyle et du fluorure d'hydrogène non transformés, éventuellement des diluants inertes, ainsi que divers sous-produits en faibles quantités, principalement un peu de 1-chloro-1-fluoroéthane et de fluorure de vinyle. Bien que l'on travaille habituellement avec un excès de fluorure d'hydrogène par rapport au chlorure de vinyle, il subsiste toujours du chlorure de vinyle non converti dans le mélange de produits réactionnels. Alors que la plupart des constituants du mélange de produits réactionnels peuvent être facilement séparés complètement par distillation, une séparation complète entre le chlorure de vinyle et le 1,1-difluoroéthane est très difficilement réalisable par distillation, ces deux composés présentant en effet des points d'ébullition relativement proches.

La présente invention a pour objet un procédé de purification du 1,1-difluoroéthane qui permette d'en éliminer efficacement le chlorure de vinyle.

L'invention concerne dès lors un procédé de purification de 1,1-difluoroéthane brut, qui se caractérise en ce qu'on met ledit 1,1-difluoroéthane brut en contact avec un charbon actif.

Par 1,1-difluoroéthane brut, on entend désigner du 1,1-difluoroéthane contaminé par du chlorure de vinyle. La teneur en chlorure de vinyle dans le 1,1-difluoroéthane brut est généralement supérieure à 20 ppm. Le plus souvent, elle dépasse 50 ppm, voire 100 ppm. En principe, le 1,1-difluoroéthane brut soumis au procédé selon l'invention peut contenir des quantités importantes de chlorure de vinyle, par exemple, de l'ordre de quelques pourcents en poids. En pratique, le 1,1-difluoroéthane brut ne contient habituellement pas plus de 10.000 ppm de chlorure de vinyle. Le 1,1-difluoroéthane brut peut en outre contenir d'autres impuretés, telles que du chlorure d'hydrogène, du fluorure d'hydrogène, du fluorure de vinyle et du 1-chloro-1-fluoroéthane. La quantité totale de ces autres impuretés ne dépasse généralement pas 1 % en poids du 1,1-difluoroéthane brut. Le plus souvent, cette quantité est inférieure à 0,5 % en poids. Ces autres impuretés qui peuvent être présentes dans le 1,1-difluoroéthane brut proviennent habituellement du procédé de fabrication du 1,1-difluoroéthane par réaction de chlorure de vinyle avec du fluorure d'hydrogène. Elles peuvent être séparées aisément du 1,1-difluoroéthane, par exemple par distillation.

Le procédé selon l'invention doit être effectué à une température adéquate, généralement d'environ -25 °C à environ +100 °C. Il est de préférence réalisé à une température d'environ -15 °C à environ +60 °C. De manière particulièrement préférée, il est réalisé à une température de -10 °C à +20 °C. Le procédé selon l'invention doit être réalisé à une pression convenable, généralement d'environ 50 kPa à environ 2000 kPa. De préférence, on travaille à une pression d'environ 100 kPa à environ 1000 kPa.

La nature du charbon actif utilisé dans le procédé selon l'invention n'apparaît pas critique. Les charbons actifs classiquement utilisés pour l'adsorption de vapeurs ou de liquides peuvent être mis en oeuvre. De bons résultats ont été obtenus dans le procédé selon l'invention avec différents charbons actifs, présentant une surface spécifique élevée, supérieure à 500 m²/g, de préférence au moins égale à 750 m²/g. Généralement, la surface spécifique du charbon actif ne dépasse pas 2000 m²/g. De bons résultats ont été obtenus avec des charbons actifs présentant une surface spécifique ne dépassant pas 1500 m²/g.

Le charbon actif est mis en oeuvre à l'état d'une poudre de particules dont la granulométrie optimum dépend des conditions dans lesquelles le procédé est mis en oeuvre. De manière générale, on sélectionne un charbon actif dont le diamètre des particules varie d'environ 0,1 mm à 10 mm. On travaille de préférence avec des particules de diamètre inférieur ou égal à 3 mm. De manière particulièrement préférée, on utilise des particules de diamètre inférieur ou égal à 1,5 mm. Par ailleurs, on préfère utiliser un charbon actif dont les particules ont un diamètre supérieur ou égal à 0,2 mm. De très bons résultats ont été obtenus avec un charbon actif de granulométrie 0,25-1 mm.

La mise en contact entre le 1,1-difluoroéthane brut et le charbon actif peut être réalisée suivant différentes techniques bien connues de l'homme du métier, dans tout appareillage adéquat. On peut opérer en lit fluidisé, mais on préfère généralement disposer le charbon actif sous la forme d'un lit fixe de particules, que l'on fait traverser par un courant du 1,1-difluoroéthane brut à épurer. Ce courant peut être liquide ou gazeux.

Lorsque l'on effectue le procédé en phase gazeuse, on réalise un temps de contact entre le 1,1-difluoroéthane brut et le charbon actif d'au moins 1 s. De préférence, on travaille avec un temps de contact supérieur à 2 s. De bons résultats ont été obtenus avec un temps de contact supérieur ou égal à environ 3 s. En principe, on peut travailler avec un temps de contact très long, par exemple de plusieurs minutes. En pratique, pour des raisons d'efficacité, on travaille généralement avec un temps de contact inférieur à 1 minute, de préférence inférieur ou égal à environ 30 s.

Lorsque l'on effectue le procédé en phase liquide, on réalise un temps de contact entre le 1,1-difluoroéthane brut et le charbon actif d'au moins environ 2 minutes. De préférence, on travaille avec un temps de contact supérieur à environ 5 minutes. En principe, on peut travailler avec un temps de contact très long, par exemple de 120 minutes. En pratique, on travaille généralement avec un temps de contact inférieur à 60 minutes, de préférence inférieur ou égal à environ 30 minutes.

Lorsque le procédé est mis en oeuvre en lit fixe, le temps de contact est défini comme le rapport du volume du lit de charbon actif au débit volumique du courant de 1,1-difluoroéthane brut. Lorsque le procédé est mis en oeuvre en lit fluidisé, le temps de contact est défini comme le rapport du volume de la cuve contenant le charbon actif au débit volumique du courant de 1,1-difluoroéthane brut.

A l'issue du procédé, le charbon actif peut être régénéré par chauffage à température modérée, par exemple de 100 à 250 °C, sous un courant gazeux, par exemple sous azote, ou sous pression réduite.

Le procédé selon l'invention s'applique à toute composition brute de 1,1-difluoroéthane contaminé par du chlorure de vinyle. Elle trouve une application particulière pour la purification du 1,1-difluoroéthane obtenu par réaction entre le chlorure de vinyle et le fluorure d'hydrogène.

Le procédé selon l'invention permet d'épurer du 1,1-difluoroéthane brut, jusqu'à obtenir une teneur résiduelle en chlorure de vinyle inférieure à 1 ppm en poids.

Les exemples suivants illustrent l'invention.

### Exemples 1 à 4

Dans une colonne en verre de 15 cm³ (longueur = 12,5 cm; diamètre = 1.25 cm), maintenue à 25°C à la pression atmosphérique, on a placé un lit de charbon actif préalablement séché à 150 °C sous courant d'azote, puis sous vide, durant 24 h. On a fait passer sur le charbon actif un courant gazeux de 1,1-difluoroéthane brut constitué d'un mélange synthétique de 1,1-difluoroéthane (HFA-152a) et de chlorure de vinyle (VC), contenant 3 050 mg de VC par kg. L'effluent épuré a été analysé par chromatographie en phase gazeuse en ligne (limite de détection du VC : 20 mg/kg) et l'essai a été poursuivi jusqu'à saturation du charbon actif en VC (teneur en VC dans le courant gazeux à l'entrée ≃ teneur en VC dans le courant gazeux à la sortie). Le débit du courant gazeux au cours de l'essai a été mesuré au moyen d'un compteur à gaz placé à la sortie de la colonne. En fin d'essai, le charbon actif a été pesé pour déterminer, par différence avec le poids du charbon actif avant essai, la quantité totale de VC et de HFA-152a adsorbée. Cette quantité exprimée en g/kg de charbon actif (C.A.) correspond à la capacité d'adsorption totale. La capacité d'adsorption en VC du charbon actif a été déterminée sur base des analyses par chromatographie en phase gazeuse réalisées en ligne. Le tableau 1 rassemble les résultats des exemples 1 à 4 réalisés à une température de 25 °C et un temps de séjour de 13 à 15 s avec 4 charbons actifs différents. Dans l'exemple 1, on a mis en oeuvre du charbon NORIT® PK de la firme NORIT. Dans les exemples 2 à 4, on a utilisé respectivement du charbon actif CALGON PCB®, du charbon actif CALGON CPG® et du charbon actif CALGON OL® de la société CALGON. Outre la capacité d'adsorption en VC et la capacité d'adsorption totale, ce tableau reprend également la teneur résiduelle minimale en VC ([VC] min) qu'il a été possible d'atteindre durant l'essai dans le courant gazeux après passage sur le charbon actif, ainsi qu'une estimation de la sélectivité d'adsorption du VC, exprimée en % et correspondant au rapport de la capacité d'adsorption en VC à la capacité d'adsorption totale (VC/totale).

**TABLEAU 1**

| Ex. | Charbon actif (C.A.) | Granulométrie (mm) | Temps de séjour (s) | [VC] min (mg/kg) | Capacité d'adsorption (g/kg C.A.) | | VC/totale (%) |
|---|---|---|---|---|---|---|---|
| | | | | | VC | totale VC+HFA-152a | |
| 1 | Norit PK | 0,25-1 | 17 | < 20 | 14.9 | 240 | 6.2 |
| 2 | Calgon PCB | 2,4-4,8 | 15 | 50 | 12 | 350 | 3.4 |
| 3 | Calgon CPG | 0,4-1,7 | 15 | 30 | 10.2 | 300 | 3.4 |
| 4 | Calgon OL | 0,3-0,85 | 13 | 40 | 10 | 320 | 3.1 |

### Exemples 5 et 6

L'exemple 1 a été répété avec des charbons actifs NORIT PK de différentes granulométries. Les résultats de ces essais, réalisés à température ambiante, sont rassemblés dans le tableau 2.

**TABLEAU 2**

| Exemple | Granulométrie (mm) | Temps de séjour (s) | [VC] min (mg/kg) | Capacité d'adsorpt. (g/kg C.A.) | |
|---|---|---|---|---|---|
| | | | | VC | Totale |
| 5 | 1 - 3 | 13 | 60-70 | 14.4 | 175 |
| 6 | 3 - 5 | 12 | 70 | 14.5 | 220 |

Les résultats repris dans le tableau 2 ne montrent aucune différence significative de capacité d'adsorption entre les différentes granulométries. Toutefois, la comparaison de ces exemples avec l'exemple 1 montre que les fines granulométries (inférieures à 1 mm) permettent d'atteindre une teneur résiduelle en VC nettement plus basse que les granulométries plus larges.

### Exemples 7 à 10

Ces exemples servent à illustrer l'influence de la température sur l'efficacité du procédé. A cet effet, l'exemple 1 a été répété à différentes températures. Les résultats de ces essais sont rassemblés dans le tableau 3.

**TABLEAU 3**

| Exemple | Température (°C) | Temps de séjour (s) | [VC] min (mg/kg) | Capacité d'adsorpt. (g/kg C.A.) | | VC/totale (%) |
|---|---|---|---|---|---|---|
| | | | | VC | Totale | |
| 7 | - 10 | 16 | < 25 | 23.4 | 270 | 11.5 |
| 8 | 0 | 14 | < 25 | 20.3 | 245 | 8.3 |
| 9 | 50 | 14 | 50 | 9.7 | 170 | 5.7 |
| 10 | 75 | 14 | 125 | 7.1 | 140 | 5.1 |

On constate qu'un abaissement de la température conduit à une augmentation de la capacité d'adsorption totale et, de manière surprenante, à une augmentation relativement plus importante de la capacité d'adsorption en VC, ce qui se traduit par une augmentation de la sélectivité d'adsorption du VC.

### Exemple 11

L'exemple 8 a été répété avec un courant gazeux de HFA-152a brut constitué d'un mélange synthétique de HFA-152a, de chlorure de vinyle (VC) et de fluorure de vinyle (VF), contenant 180 mg de VC et 490 mg de VF par kilo de mélange. L'effluent épuré a été analysé par chromatographie en phase gazeuse en ligne, avec une limite de détection du VC de 1 mg/kg.

Dans ces conditions, une teneur résiduelle minimale en VC ([VC] min) inférieure à 1 mg/kg a été atteinte, alors que le VF n'est pas adsorbé sélectivement par rapport au HFA-152a.

## Revendications

1. Procédé de purification de 1,1-difluoroéthane brut en chlorure de vinyle caractérisé en ce qu'on met ledit 1,1-difluoroéthane brut en contact avec un charbon actif.

2. Procédé selon la revendication 1, caractérisé en ce que le 1,1-difluoroéthane brut contient de 100 à 10.000 ppm en poids de chlorure de vinyle.

3. Procédé selon la revendication 1 ou 2, caractérisé en ce qu'on opère à une température de -25 °C à +100 °C.

4. Procédé selon la revendication 3, caractérisé en ce qu'on opère à une température au maximum égale à 60 °C.

5. Procédé selon l'une quelconque des revendications 1 à 4, caractérisé en ce qu'on opère à une pression de 50 kPa à 2000 kPa.

6. Procédé selon l'une quelconque des revendications 1 à 5, caractérisé en ce qu'on dispose le charbon actif sous la forme d'un lit fixe de particules, que l'on fait traverser par le 1,1-difluoroéthane brut.

7. Procédé selon l'une quelconque des revendications 1 à 6, caractérisé en ce qu'on met le 1,1-difluoroéthane brut à l'état liquide en contact avec le charbon actif pendant un temps de 2 minutes à 120 minutes.

8. Procédé selon l'une quelconque des revendications 1 à 6, caractérisé en ce qu'on met le 1,1-difluoroéthane brut à l'état gazeux en contact avec le charbon actif pendant un temps de 3 s à 30 s.

9. Procédé selon l'une quelconque des revendications 1 à 8, caractérisé en ce qu'on sélectionne un charbon actif dont le diamètre des particules varie de 0,2 à 3 mm.

10. Procédé selon l'une quelconque des revendications 1 à 9 caractérisé en ce que le 1,1-difluoroéthane brut provient de la réaction entre du chlorure de vinyle et du fluorure d'hydrogène.

## Claims

1. Process for the purification of crude 1,1-difluoroethane from vinyl chloride, characterized in that the said crude 1,1-difluoroethane is brought into contact with an active carbon.

2. Process according to Claim 1, characterized in that the crude 1,1-difluoroethane contains from 100 to 10,000 ppm by weight of vinyl chloride.

3. Process according to Claim 1 or 2, characterized in that the process is carried out at a temperature from - 25 °C to + 100 °C.

4. Process according to Claim 3, characterized in that the process is carried out at a temperature at most equal to 60 °C.

5. Process according to any one of Claims 1 to 4, characterized in that the process is carried out at a pressure from 50 kPa to 2000 kPa.

6. Process according to any one of Claims 1 to 5, characterized in that the active carbon is arranged in the form of a stationary bed of particles, through which the crude 1,1-difluoroethane is made to pass.

7. Process according to any one of Claims 1 to 6, characterized in that the crude 1,1-difluoroethane in the liquid form is brought into contact with the active carbon for a time from 2 minutes to 120 minutes.

8. Process according to any one of Claims 1 to 6, characterized in that the crude 1,1-difluoroethane in the gaseous state is brought into contact with the active carbon for a time from 3 s to 30 s.

9. Process according to any one of Claims 1 to 8, characterized in that an active carbon is selected in which the diameter of the particles varies from 0.2 to 3 mm.

10. Process according to any one of Claims 1 to 9, characterized in that the crude 1,1-difluoroethane is obtained by the reaction between vinyl chloride and hydrogen fluoride.

## Patentansprüche

1. Verfahren zur Reinigung von rohem 1,1-Difluorethan von Vinylchlorid, dadurch gekennzeichnet, daß man das rohe 1,1-Difluorethan mit einer Aktivkohle in Kontakt bringt.

2. Verfahren nach Anspruch 1, dadurch gekennzeichnet, daß das rohe 1,1-Difluorethan von 100 bis 10.000 ppm des Gewichts an Vinylchlorid enthält.

3. Verfahren nach Anspruch 1 oder 2, dadurch gekennzeichnet, daß man bei einer Temperatur von -25°C bis +100°C arbeitet.

4. Verfahren nach Anspruch 3, dadurch gekennzeichnet, daß man bei einer Temperatur von maximal etwa bis 60°C arbeitet.

5. Verfahren nach einem der Ansprüche 1 bis 4, dadurch gekennzeichnet, daß man bei einem Druck von 50 kPa bis 2.000 kPa arbeitet.

6. Verfahren nach einem der Ansprüche 1 bis 5, dadurch gekennzeichnet, daß man die Aktivkohle in Form eines festen Betts der Teilchen zur Verfügung stellt, welches man von dem rohen 1,1-Difluorethan durchdringen läßt.

7. Verfahren nach einem der Ansprüche 1 bis 6, dadurch gekennzeichnet, daß man das rohe 1,1-Difluorethan in flüssigem Zustand während eines Zeitraums von zwei Minuten bis 120 Minuten mit der Aktivkohle in Kontakt bringt.

8. Verfahren nach einem der Ansprüche 1 bis 6, dadurch gekennzeichnet, daß man das rohe 1,1-Difluorethan in gasförmigem Zustand während eines Zeitraums von 3 s bis 30 s mit der Aktivkohle in Kontakt bringt.

9. Verfahren nach einem der Ansprüche 1 bis 8, dadurch gekennzeichnet, daß man eine Aktivkohle auswählt, deren Teilchendurchmesser von 0,2 bis 3 mm variiert.

10. Verfahren nach einem der Ansprüche 1 bis 9, dadurch gekennzeichnet, daß das rohe 1,1-Difluorethan aus der Reaktion zwischen Vinylchlorid und Fluorwasserstoff stammt.
